# EUROPEAN PATENT APPLICATION

(11) **EP 4 027 261 A1**
(43) Date of publication of application: **13.07.2022**
(21) Application number: 20861018.8
(22) Date of filing: 20.08.2020
(51) Int. Cl.: G06F 30/10

(54) **MATERIAL PROPERTIES PREDICTION SYSTEM AND INFORMATION PROCESSING METHOD**

(30) Priority: 05.09.2019 JP 2019162137
(71) Applicant: Hitachi, Ltd., Tokyo 100-8280 (JP)
(72) Inventor: ASAHARA, Akinori, Tokyo 100-8280 (JP); HAYASHI, Takayuki, Tokyo 100-8280 (JP); KANAZAWA, Takuya, Tokyo 100-8280 (JP); MORITA, Hidekazu, Tokyo 100-8280 (JP)
(74) Representative: Mewburn Ellis LLP
(86) International application number: PCT/JP2020/031426
(87) International publication number: WO 2021/044857

(57) **Abstract**

The present invention defines a spatial structure of a molecule without allowing freedom with respect to the selection of a coordinate system and predicts material properties based on a spatial structure of the molecule. A material properties prediction system that predicts properties of a material includes a three-dimensional molecular structure calculation unit having a function of calculating positional coordinates of atoms constituting a molecule from a structural formula of the material; a spatial structure feature quantity calculation unit having a function of selecting three atoms to form a triangle based on the position coordinates of atoms calculated by the three-dimensional molecular structure calculation unit, and calculating, as a spatial structure feature quantity, distances between the three atoms and another atom; and a material properties prediction unit that predicts material properties using, as an explanatory variable, the spatial structure feature quantity generated by the spatial structure feature quantity calculation unit.

## Description

### Technical Field

The present invention relates to a technique for supporting an experiment in materials science and the like.

### Background Art

With the development of statistical processing technologies relating to data analysis, there is an increasing demand for data analysis in materials science as well. In particular, in the field of materials science, a method that is called screening is known in which a candidate for a next experiment is selected based on known data in order to efficiently develop a new material.

As a screening method, various experimental data is input to an information system, machine learning is performed to build a model that predicts experimental results, and screening is performed based on the prediction performed by the model. For this prediction, a method is known, which uses various parameters relating to material design as arguments to perform regression analysis to obtain a function of returning material properties.

### Citation List

### Patent Literature

PTL 1: Japanese Patent Application Laid-Open No. 2004-086892
PTL 2: Japanese Unexamined Patent Application Publication (Translation of PCT Application) No. 2017-520868
PTL 3: Japanese Patent Application Laid-Open No. 2008-081435

### Summary of Invention

### Technical Problem

In material development, it is expected that it will be possible to more accurately identify a promising potential for a candidate for a new material by improving the accuracy of predicting material properties and efficiently develop a material by omitting unnecessary experiments.

In regression analysis, a variable corresponding to an argument of a function is called an explanatory variable, and a value corresponding to a return value of the function is called an objective variable. In material properties prediction, properties of a material are used as an objective variable, and an explanatory variable that indicates a feature of the material is selected such that the material properties can be predicted. Since the accuracy of the prediction varies depending on the selection of the explanatory variable. Therefore, it is important to prepare a variety of methods for generating an explanatory variable such that the methods can be used to predict various material properties.

In Patent Literature 1, material properties are predicted using a mixing ratio of components as an explanatory variable. This method can be used to predict properties of a material obtained by mixing a plurality of substances. However, this method is suitable for predicting properties of a single substance.

Patent Literature 2 discloses a method for dividing a space around a molecule into spatial lattices (voxels), expressing the three-dimensional structure of the molecule using the number of atoms in each voxel, and using it as an explanatory variable. According to this method, it is possible to predict material properties based on the three-dimensional shape of a single molecule.

However, in the method using voxels, there is a degree of freedom in how to determine a coordinate system. That is, there is no method of determining where the origin needs to be placed in a molecule, determining which direction an x axis needs to be, and the like. In other words, many voxels may be present in the same substance.

In the invention described in Patent Literature 2, although the freedom is tried to be incorporated into regression analysis by generating a large amount of data with different origins and different angles, a large amount of duplicate data is input, and a calculation time and the like will increase significantly. In addition, depending on a technique for the regression analysis, it is not clear whether a regression analysis algorithm can appropriately incorporate the freedom into a model, and there remains a concern that the accuracy of prediction will be rather reduced. In addition, even when the prediction can be performed well, there is a problem that reverse calculation cannot be performed. For example, when a condition under which the highest predicted value of properties of a material is obtained needs to be found, it may be sufficient to search for the maximum value of a function that returns the properties of the material. However, even when explanatory variables of voxels at that time are obtained, the structure of a corresponding molecule cannot be easily inferred.

As a screening method based on the three-dimensional structure of a molecule, a method for evaluating a similarity with a known molecule as disclosed in Patent Literature 3 is also known. Since this method is based on another molecule, the effect of the freedom of the coordinate system of a molecule alone is small, but there is a problem that inverse calculation is still difficult and the method cannot be applied unless a sufficient number of molecules are known.

Therefore, it is desirable to define the spatial structure of a molecule without allowing freedom with respect to the selection of a coordinate system, and to predict material properties based on the three-dimensional structure of the molecule.

### Solution to Problems

According to a preferable aspect of the present invention, a material properties prediction system that predicts properties of a material includes a three-dimensional molecular structure calculation unit having a function of calculating positional coordinates of atoms constituting a molecule from a structural formula of the material; a spatial structure feature quantity calculation unit having a function of selecting three atoms to form a triangle based on the positional coordinates of the atoms calculated by the three-dimensional molecular structure calculation unit and calculating, as a spatial structure feature quantity, distances between the three atoms and another atom; and a material properties prediction unit that predicts the material properties using, as an explanatory variable, the spatial structure feature quantity generated by the spatial structure feature quantity calculation unit.

According to another preferable aspect of the present invention, an information processing method includes performing a three-dimensional molecular structure calculation process of receiving a structural formula of a material and calculating positions of atoms constituting a molecule from the structural formula of the material; and performing a spatial structure feature quantity calculation process of selecting three atoms to form a triangle based on the calculated positions of the atoms, and calculating distances between the three atoms and another atom to obtain a spatial structure feature quantity.

### Advantageous Effects of Invention

It is possible to define the spatial structure of a molecule without allowing freedom with respect to the selection of a coordinate system and to predict material properties based on the three-dimensional structure of the molecule.

### Brief Description of Drawings

Figure 1 is a functional block diagram illustrating an example of a schematic configuration according to Example 1.
Figure 2 is a block diagram illustrating an example of a configuration of physical implementation according to Example 1.
Figure 3 is a schematic diagram illustrating an example of a usage procedure according to Example 1.
Figure 4 is a flow diagram illustrating an example of a material DB update process according to Example 1.
Figure 5 is an image diagram illustrating an example of a display screen for receiving experimental data according to Example 1.
Figure 6 is a table diagram illustrating an example of a configuration of the experimental data according to Example 1.
Figure 7 is a table diagram illustrating an example of an experimental data table of a material DB according to Example 1.
Figure 8 is a table diagram illustrating an example of information corresponding to a single molecule and included in three-dimensional molecular structure data according to Example 1.
Figure 9 is a schematic diagram of a three-dimensional molecular structure assumed in Example 1.
Figure 10 is a flow diagram illustrating an example of a spatial structure feature quantity calculation process according to Example 1.
Figure 11 is a schematic diagram of a process of calculating a spatial structure feature quantity assumed in Example 1.
Figure 12 is a table diagram illustrating an example of a table of a spatial structure feature quantity according to Example 1.
Figure 13 is a flow diagram illustrating an example of a material properties prediction process according to Example 1.
Figure 14 is an image diagram illustrating an example of a material properties prediction display according to Example 1.
Figure 15 is a table diagram illustrating an example of a configuration of data for material properties prediction according to Example 1.
Figure 16 is a functional block diagram illustrating an example of a schematic configuration according to Example 2.
Figure 17 is a flow diagram illustrating an example of a material properties prediction process according to Example 2.
Figure 18 is an image diagram illustrating an example of a material properties prediction display according to Example 2.

### Description of Embodiments

Embodiments are described in detail with reference to the drawings. However, the present invention is not construed as being limited to the following description of the embodiments. It is easily understood by those skilled in the art that a specific configuration thereof can be modified without departing from the idea or gist of the present invention.

For configurations according to the present invention described below, the same reference signs are used in common for the same components or components having the same functions between different drawings, and a duplicate description may be omitted.

When multiple elements having the same function or having similar functions are present, they may be explained by adding different subscripts to the same reference sign. However, when it is not necessary to distinguish between the multiple elements, the subscripts may be omitted for explanation.

Notations such as "first", "second", and "third" in the present specification and the like are added to identify components, and do not necessarily limit the number, order, or contents thereof. In addition, numbers for identifying components are used for each context, and numbers used in one context do not necessarily indicate the same configuration in other contexts. Furthermore, this does not prevent a component identified by a certain number from functioning as a component identified by another number.

Positions, sizes, shapes, ranges, and the like of configurations illustrated in the drawings and the like may not represent the actual positions, sizes, shapes, ranges, and the like in order to facilitate understanding of the present invention. Therefore, the present invention is not necessarily limited to the positions, the sizes, the shapes, the ranges, and the like disclosed in the drawings and the like.

### [Example 1]

### <1. System Configuration>

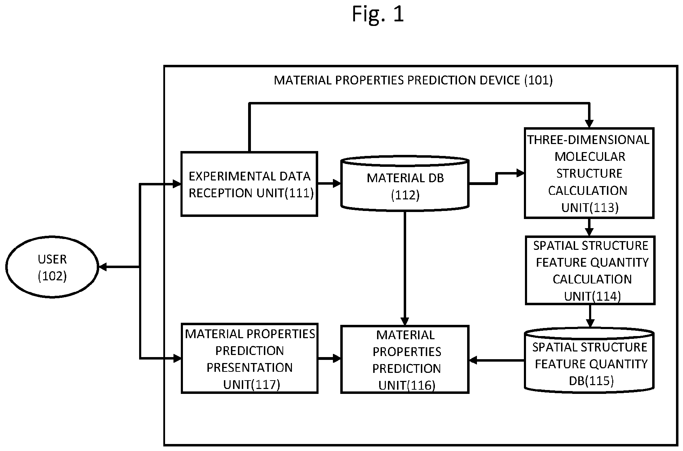

Figure 1 illustrates an example of a material properties prediction device according to Example 1. The material properties prediction device (101) according to the present example is a device that receives an operation of a user (102), and is a system that includes an experimental data reception unit (111) that receives experimental data from the user, a material database (DB) (112) in which features and properties of a material are stored, a three-dimensional molecular structure calculation unit (113) that receives information of a structural formula of a molecule of the material and estimates coordinates of atoms constituting the molecule, a spatial structure feature quantity calculation unit (114) that calculates a spatial structure feature quantity of the molecule based on the atoms' positions calculated by the three-dimensional molecular structure calculation unit (113), a spatial structure feature quantity DB (115) that stores the spatial structure feature quantity calculated by the spatial structure feature quantity calculation unit (114) in association with an identifier of the molecule, a material properties prediction unit (116) that predicts material properties of the molecule that are not measured from information of the molecule whose material properties value has been measured based on the information stored in the material DB (112) and the spatial structure feature quantity DB (115), and a material properties prediction presentation unit (118) that presents the results of the material properties prediction unit (116) to the user (102).

In the present example, the material properties prediction device (101) is constituted by an information processing device such as a server including an input device, an output device, a storage device, and a processing device. Functions such as calculation and control are implemented by executing a program stored in the storage device by the processing device to cause a predetermined process and other hardware to collaborate with each other. Figure 1 illustrates functional blocks instead of a hardware configuration of the information processing device. A program to be executed as each of the functional blocks by a calculator or the like, a function thereof, or means for enabling the function may be referred to as "function", "means", "section", "unit", "module", or the like.

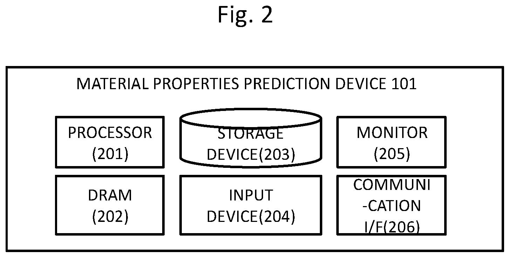

Figure 2 illustrates an example of a configuration of physical implementation according to Example 1. The material properties prediction device (101) can be implemented using a general computer. That is, the material properties prediction device (101) is a device that includes a processor (201) with computing performance, a dynamic random access memory (DRAM) (202) that is a highspeed readable and writable volatile temporal storage region, a storage device (203) that uses an HDD (magnetic disk drive), a flash memory, or the like and is a permanent storage region, an input device (204) that is to be operated and is a mouse, a keyboard, or the like, a monitor (205) that presents an operation to the user, and an interface (206) that is provided for communication with an external and is a serial port or the like.

The experimental data reception unit (111), the three-dimensional molecular structure calculation unit (113), the spatial structure feature quantity calculation unit (114), the material properties prediction unit (116), and the material properties prediction presentation unit (118), which are illustrated in Figure 1, can be implemented by causing the processor (201) to execute a program stored in the storage device (203). The material DB (112) and the spatial structure feature quantity DB (115) can be implemented by causing the processor (201) to execute a program for accumulating data in the storage device (203).

The configuration illustrated in Figure 2 may be included in a single computer, or any portion of the configuration may be included in another computer connected to a network. That is, a plurality of computers may constitute a similar system.

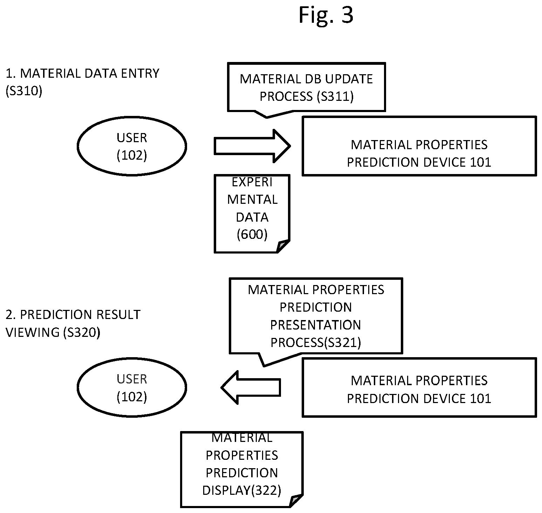

Figure 3 schematically illustrates a usage procedure of the system according to Example 1. In Example 1, two procedures, which are a material data entry (S310) for entering data on material properties prediction by the user and prediction result viewing (S320) for confirming a result of the material properties prediction, are performed.

The material data entry (S310) is a procedure for entering, in the material properties prediction device (101), experimental data (600) that is a data set storing data of a material for which an experiment has been conducted and data of a material for which an experiment will be conducted. The material properties prediction device (101) performs a material DB update process (S311) based on the data to update information stored in the material DB (112).

In the prediction result viewing (S320), the material properties prediction device (101) executes a material properties prediction presentation process (S321) in accordance with a request from the user (102) to present a material properties prediction display (322) that is a screen obtained by visualizing the result of the material properties prediction.

### <2. Material Data Entry Process>

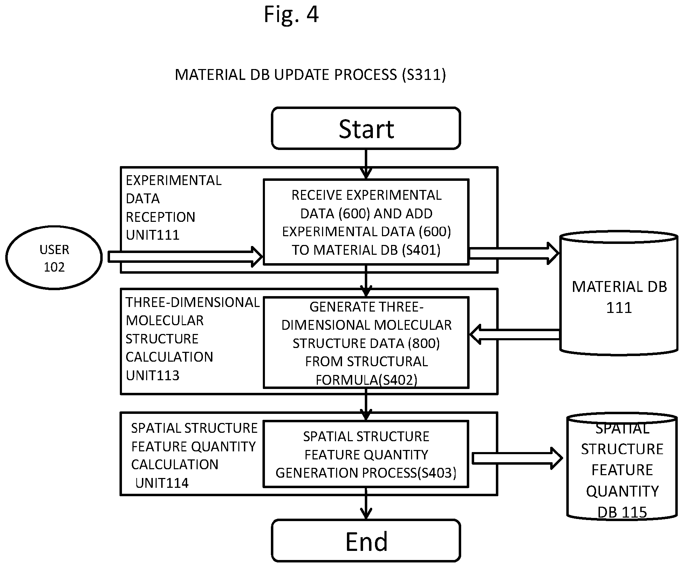

Figure 4 illustrates an example of a procedure for the material DB update process (S311). In the material DB update process (S311), first, the experimental data reception unit (111) receives the experimental data (600) from the user (102) and updates the material DB (112) (S401). After that, the three-dimensional molecular structure calculation unit (113) generates three-dimensional molecular structure data (800) corresponding to data of the material DB (112) (S402). The spatial structure feature quantity calculation unit (114) performs a spatial structure feature quantity generation process (S403) using the three-dimensional molecular structure data (800) to calculate a spatial structure feature quantity (1100) and stores the spatial structure feature quantity (1100) to the spatial structure feature quantity DB (115).

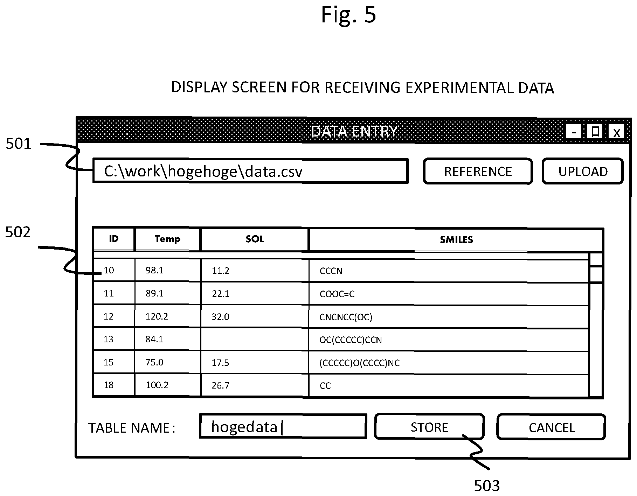

Figure 5 illustrates an example of a screen displayed on the monitor (205) to receive the experimental data (600) from the user (102) in the first step (S401) of the material DB update process (S311). In Example 1, the user (102) stores the experimental data to a file in advance and gives the experimental data (600) in a form in which the position of the file is specified in a text box (501). The data in a table format that is the known Comma Separated Value (CVS) format is described in the given file, and a result obtained by interpreting the data and formed in a table format is displayed on a table screen (502).

Figure 5 exemplifies an "ID" that is an experiment identifier that is information described, "Temp" indicating a temperature at the time of the experiment, "SOL" indicating water solubility at that time, and a character string "SMILES" indicating the structural formula of a material. In this example, water solubility is material properties to be predicted, and data with a blank in an SOL field indicates a condition for an experiment not conducted. The giving of the data is an example. Another method may be used when the experimental data including the structural formula of the material and the material properties can be given as information that can be converted into a table format. The information is displayed on the table screen (502) and stored in the material DB (112) by a button (503).

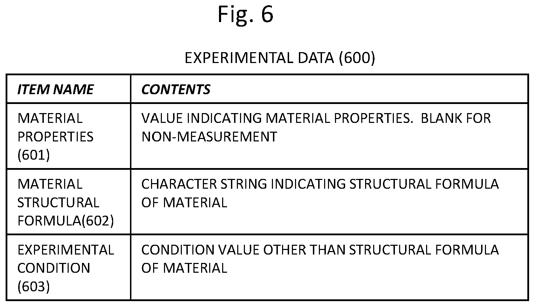

Figure 6 illustrates an example of a configuration of one record of the experimental data (600). In this example, the experimental data (600) is information including, as one record, information of material properties (601), a material structural formula (602) that is information indicating the structure formula of a material in the SMILES format or the like, and an experimental condition (603) indicating a condition at the time of an experiment, such as a temperature, pressure, or the like. The experimental data (600) includes a plurality of such records. The information is associated with each item of the table screen (502) illustrated in Figure 5. In the present example, each item can be determined based on which element is associated with the item or based on association with a predetermined item name. The association relationships may be entered on the screen by the user (102). In addition, in the material properties (601), a value found by the experiment or the like is stored or a blank is stored when an experiment is not conducted. Definitions and numbers of the material properties (601) and the experimental conditions (603) are arbitrary.

In the first step (S401) of the material DB update process (S311) illustrated in Figure 4, the experimental data (600) is interpreted and formatted and is stored as an experimental data table of the material DB (112).

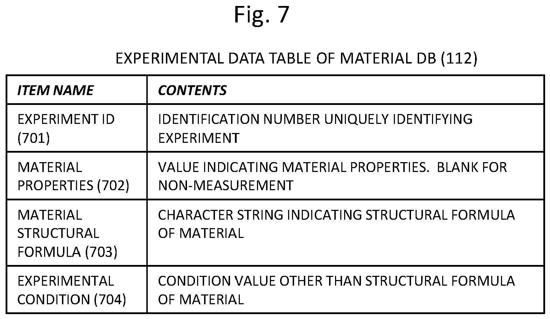

Figure 7 illustrates information of one record of the experimental data table. This data includes an experiment ID (701) that is a serial number or the like uniquely identifying an experiment, material properties (702) derived from the material properties (601) of the experimental data (600), a material structural formula (703) derived from the material structural formula (602) of the experimental data (600), and an experimental condition (704) derived from the experimental condition (603). These items may be obtained by converting the information serving as the sources of the derivation into information having a unified unit in a unified format.

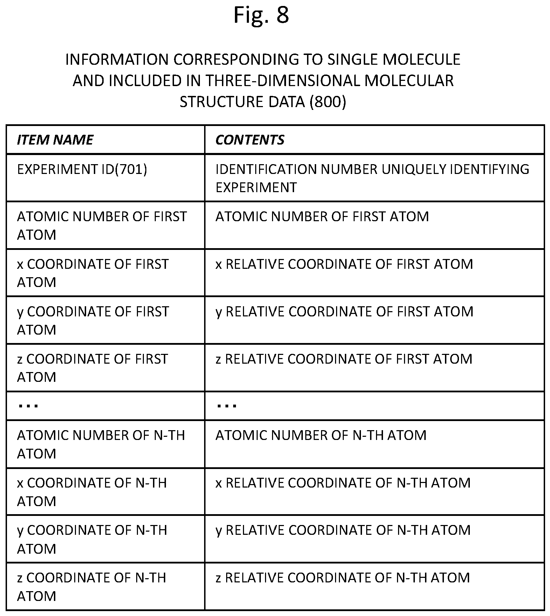

Figure 8 illustrates a configuration of three-dimensional molecular structure data (800) corresponding to a single molecule and calculated by the three-dimensional molecular structure calculation unit (113) in the process (S402) that is the second step of the material DB update process (S311). This data is information in which relative coordinates of atoms constituting the molecule are described. The number of atoms constituting a molecule varies depending on the type of the molecule. Therefore, in this case, the molecule with a number N of atoms is exemplified. The relative coordinates are coordinate values that can be simply calculated by a known method such as a distance geometry method from information of atoms expressed in the material structural formula (703) and bonding of the atoms. Normally, when a distance between atoms is smaller than the Van der Waals radius, the atoms correspond to a state in which the atoms are bonded.

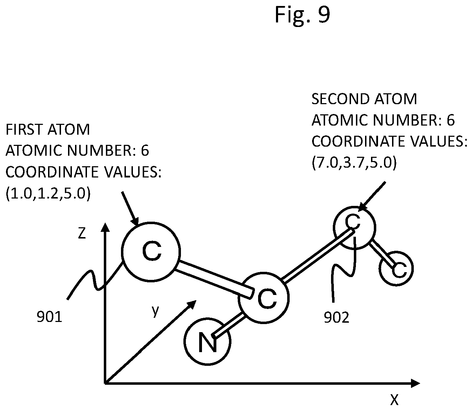

Figure 9 schematically illustrates an example of the three-dimensional molecular structure. In this drawing, the position of a certain one molecule in a three-dimensional space is illustrated, spheres with element symbols indicate atoms, and lines between the spheres indicate bonding (hydrogen atoms are omitted). Although types of the atoms constituting the molecule and information of the bonding are described in the structural formula, the positions of the atoms are not normally described. For example, information indicating that a carbon atom (901) is present and information on another carbon atom bonded are described in the structural formula.

However, for example, coordinate values (1.0, 1.2, 5.0) of the carbon atom (901) and coordinate values (7.0, 3.7, 5.0) of a carbon atom (902) are not described and thus need to be calculated. In an example of a known method, an atom is once placed at a position in the van der Waals radius or the like, the position is optimized and calculated such that a bonding angle and the like are appropriate values. For this calculation method, there are various known methods. Therefore, the calculation may be performed using any of the known methods as long as a certain degree of accuracy can be obtained.

The coordinate values obtained as results of this calculation are relative coordinates, and the coordinate system varies depending on the molecule. For this, there is a method of creating a certain unified standard by using the center of gravity of the molecule or the like. However, the present example has an advantage that this standard for a coordinate system may not be required and any standard may be used.

For each material structural formula (703) of the experimental data table, the positions of atoms are calculated and three-dimensional molecular structure data (800) is obtained as a result in which the positions are described in appropriate order. In this case, a corresponding experiment ID (701) needs to be associated with the experimental data (600).

The third step of the material DB update process (S311) is the spatial structure feature quantity generation process (S403) of calculating a feature quantity from the three-dimensional molecular structure data (800).

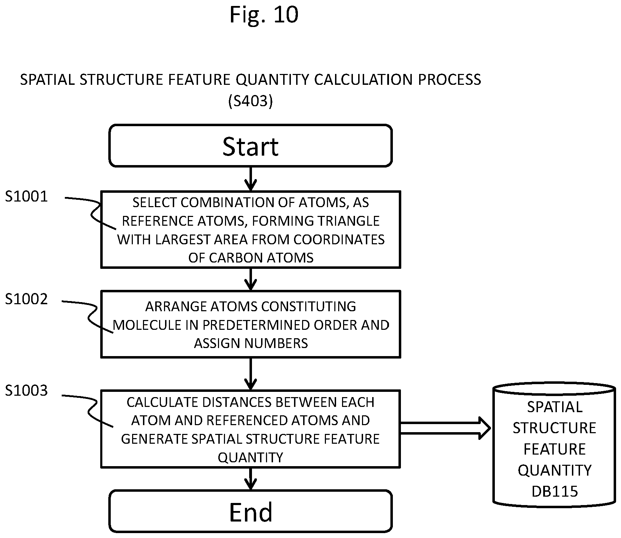

Figure 10 illustrates this process in detail. In this process, first, three atoms serving as a standard in a three-dimensional structure are selected (S1001). In the present example, combinations of three carbon atoms constituting a molecule are checked and atoms forming a triangle with the largest area are selected and used, but atoms suitable for expressing a feature of the molecule can be selected by using another standard. For example, values relating to important positions as a mass distribution of the inside of the molecule can be calculated by adding an element having a large molecular weight other than carbon atoms to candidates or the like.

In the present example, carbon atoms are prioritized as the atoms forming the triangle. This is due to the fact that, when the material is organic, the basis of the structure is carbon atoms. It is not essential to select a carbon atom, and atoms that cause the accuracy to be high may be selected as appropriate. In fact, since it is inferred that the atoms to be used vary depending on properties to be predicted, it is desirable that the user can configure a setting as appropriate.

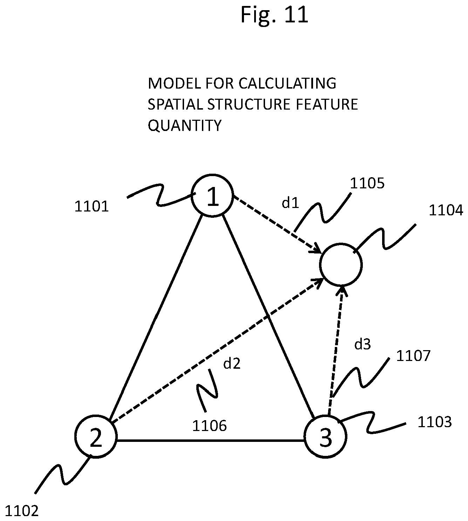

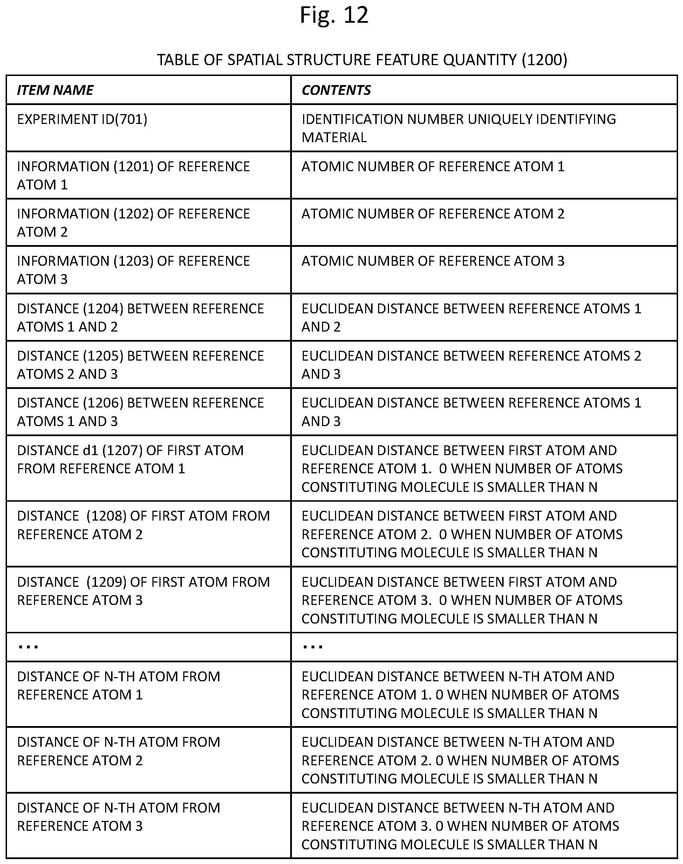

A circulation direction is defined by adding reference atomic numbers (reference numbers) or the like for the three atoms forming the triangle serving as the standard in the three-dimensional structure, as described later with reference to Figures 11 and 12. In Figure 11, an atom 1101 has a reference atomic number 1, an atom 1102 has a reference atomic number 2, and an atom 1103 has a reference atomic number 3.

As described above, since a method for selecting atoms forming a triangle may vary depending on material properties to be predicted, atoms that cause the highest accuracy may be selected via calculation of a plurality of combinations.

Next, other atoms are rearranged in accordance with a predetermined standard (S1002). In this case, as this standard, the shortest linear distances from the center of gravity of the triangle formed by the reference atoms are calculated and the atoms are arranged in the order from the shortest distance. Alternatively, the atoms can be arbitrarily arranged based on the order determined based on only relative distances between the atoms. Identification numbers are assigned to the other atoms based on the arrangement order.

Next, linear distances between the atoms and the three reference atoms are calculated and used as a feature quantity (S1003). As described above, the foregoing linear distances can be long and an error can be reduced by using, as the reference atoms, atoms forming a triangle with the largest area.

Figure 11 illustrates a schematic diagram. The drawing illustrates three reference atoms (1101), (1102), and (1103) and arrows (1105), (1106), and (1107) between the reference atoms and another atom (1104). Lengths of the arrows are illustrated as d1, d2, and d3. When the three lengths are determined, coordinates of the atom (1104) are uniquely determined, except for the reverse of the front and back surfaces of the triangle formed by the three reference atoms. Conversely, when the coordinates are determined, the lengths of the three arrows (1105), (1106), and (1107) are determined. That is, there is a one-to-one relationship, except for mirror image symmetry.

When signs of d1, d2, and d3 are determined to be positive in the case where the circulation direction of the triangle formed by the three reference atoms (1101), (1102), and (1103) is the clockwise direction as viewed from the target atom (1104) side, and are determined to be negative in the case where the circulation direction is the counterclockwise direction as in Figure 11, relative positional relationships of the atoms are uniquely described.

In the present example, these values are arranged in a row to indicate a spatial structure feature quantity indicating the spatial structure of the molecule. These values do not have dependency on the orientation of the coordinate system and the position of the origin that relate to the coordinate values of the atoms within the molecule. In addition, these values have a feature suitable for material properties prediction in which the molecular structure can be reversely calculated from the values when the values are determined.

Figure 12 illustrates a table of the spatial structure feature quantity. The calculated spatial structure feature quantity is stored in the spatial structure feature quantity DB (115) in a format illustrated in Figure 12. For the storage in the spatial structure feature quantity DB (115), since a record that is included in the experimental data table of the material DB (112) and from which the data is derived is recorded, an experiment ID (701) is added. For this, a method for reproducing associations based on the arrangement order or the like may be used. In addition, since information of the triangle formed by the reference atoms is recorded, the atomic numbers (1201), (1202), and (1203) of the reference atoms and distances (1204), (1205), and (1206) between the reference atoms are recorded.

After that, as the foregoing spatial structure feature quantity, distances between each atom and the reference atoms are described (1207), (1208), and (1209). In this case, items are created such that the number of items is based on a case where the number of atoms is the largest among cases stored in the material DB (112), and 0 is added to an item in which an atom corresponding to a molecule that does not have the maximum number of atoms is not present. In this case, distances may be expressed in any unit. However, Angstrom is used for the distances in this example.

By the foregoing process, new experimental data can be added to the material DB (112). That is, the procedure for the material data entry (S310) is completed.

### <3. Prediction Result Viewing Process>

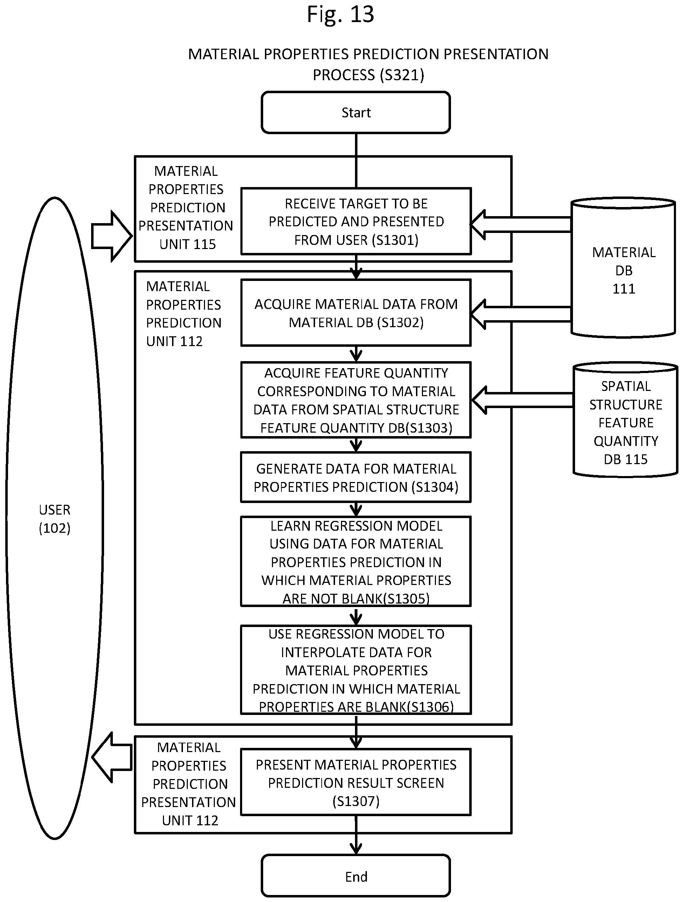

The material properties prediction presentation process (S321) in the prediction result viewing (S320) is described using Figure 13. First, a material identification prediction presentation unit (117) uses the monitor (205) to present the material properties prediction display (322) to the user (102) and receives the designation of an experimental data table targeted for prediction (S1301). In this case, contents of the experimental data table stored in the material DB (112) may be used.

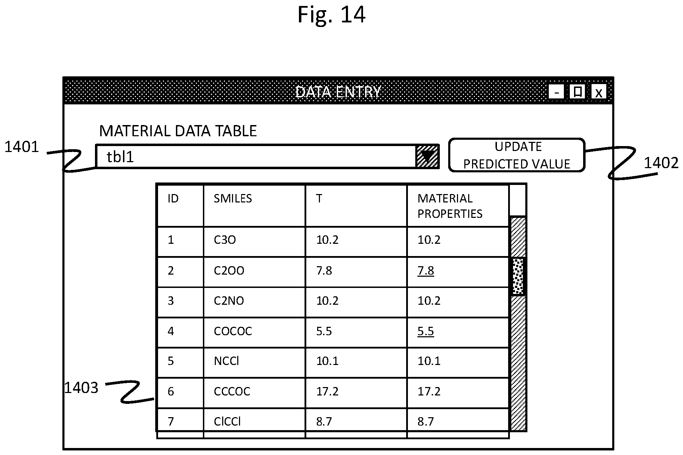

Figure 14 illustrates an example thereof. In a drop-down box (1401) illustrated in Figure 14, for example, a file name of the experimental data table is displayed as a candidate. When a button (1402) for updating a predicted value is pressed, the material identification prediction presentation unit (117) transmits, to the material properties prediction unit (116), an instruction to use a predicted value to perform interpolation on a record that is included in the experimental data table (Figure 7) and in which material properties (702) are blank, and a result thereof is displayed on a screen (1403). In Figure 14, underlined values indicate that blank data has been interpolated.

Upon receiving the instruction to perform the interpolation from the material identification prediction presentation unit (117), the material properties prediction unit (116) acquires data of the designated experimental data table from the material DB (112) (S1302) and uses the experiment ID (701) thereof to acquire a corresponding record from the spatial structure feature quantity table (1200) (S1303). The material properties prediction unit (116) associates the data with the record, thereby generating data to be used for material properties prediction (S1304).

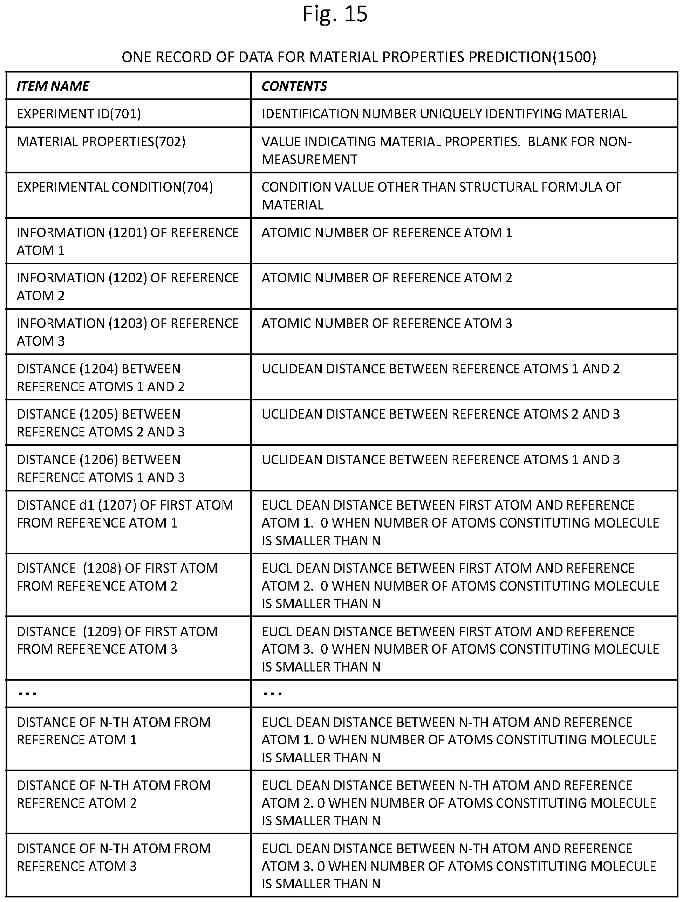

Figure 15 illustrates a configuration (1500) of one record of data for material properties prediction. This data is obtained by acquiring, from the material DB (112), the experiment ID (701), the material properties (702), and the experimental condition (704), acquiring, from the spatial structure feature quantity DB (115), the information (1201) of the reference atom 1, the information (1202) of the reference atom 2, the information (1203) of the reference atom 3, the distance (1204) between the reference atoms 1 and 2, the distance (1205) between the reference atoms 2 and 3, the distance (1206) between the reference atoms 1 and 3, the distance d1 (1207) between each atom and the reference atom 1, the distance (1208) between each atom and the reference atom 2, and the distance (1209) between each atom and the reference atom 3, and combining records including the common experiment ID (701) .

The material properties prediction unit (116) removes, from the data for material properties prediction, a record that has material properties (702) not measured or in which the material properties (702) are blank, sets the items excluding the experiment ID (701) and the material properties (702) as explanatory variables, sets the material properties (702) as an objective variable, and performs known regression analysis to obtain a prediction function (S1305). This procedure means that, when the prediction function is expressed as y = f(x), y is an objective variable, x is an explanatory variable, and the function form of y is defined such that y can be predicted when x is determined. After generating a regression model, the material properties prediction unit (116) selects data with material properties (702) that are not measured or are blank, and uses the foregoing prediction function of y = f(x) to calculate a predicted value of the material properties (702) (S1306).

As a method to be used to build the prediction function f, a known multivariate regression analysis method can be used. For example, a known high-precision nonlinear regression method, such as a regression tree, random forests, support vector regression, Gaussian process regression, or a neural network, can be used as long as the method is a regression analysis method that uses multivariate as an argument. As described above, this prediction result is reflected in the screen (1403) by the material identification prediction presentation unit (117) (S1307). In the present example, although only the spatial structure feature quantity and the experimental condition are used as the explanatory variables, a certain amount (for example, a molecular weight or an electric charge) may be calculated and used in fact. In addition, like a known recursive neural network, when a technique capable of performing prediction using sequential information is used, it may be possible to perform the prediction without using data in which the distance d1 (1207) between each atom and the reference atom 1 is 0, and high accuracy may be obtained.

In the foregoing example, it is possible to incorporate the spatial structure of a molecule into prediction to perform evaluation without performing special post-processing on the spatial structure for screening of experimental design. Therefore, it is expected to improve the accuracy of prediction.

### [Example 2]

Example 2 has a feature in which not only a predicted value of material properties not measured is calculated, but also a condition under which optimal material properties are predicted is searched, displayed on a screen, and used to make experimental design.

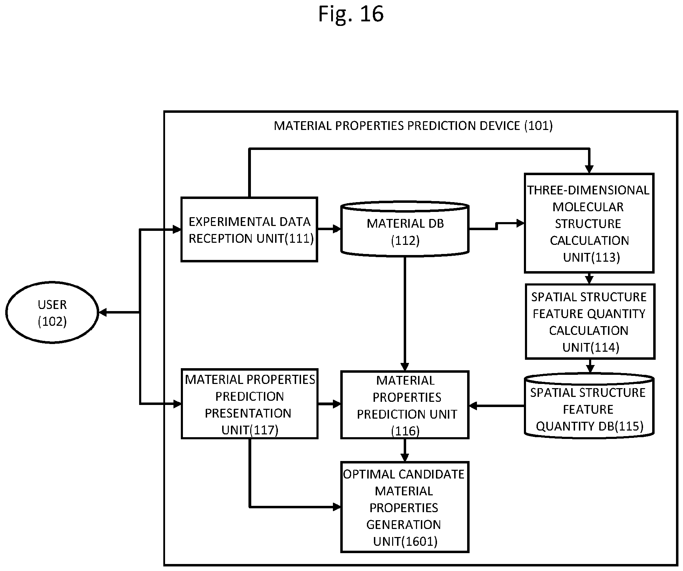

Figure 16 illustrates an example of a configuration according to Example 2. Example 2 is different from Example 1 in an optimal candidate material properties generation unit (1601). The optimal candidate material properties generation unit (1601) has a function of searching for a condition under which optimal material properties are predicted. The optimal candidate material properties generation unit (1601) can three-dimensionally display a virtual molecular structure, which is a candidate for the optimum value, on the material properties prediction display (322) presented to the user in the material properties prediction presentation process (S321) described in Example 1.

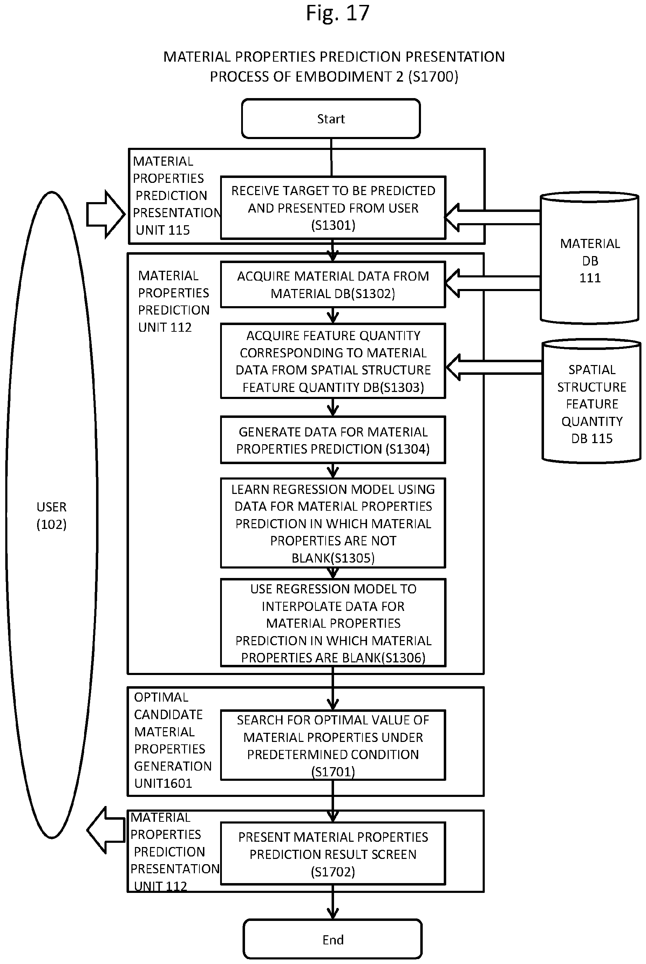

Figure 17 illustrates a material properties prediction presentation process (S1700) according to Example 2 using the optimal candidate material properties generation unit (1601). The material properties prediction presentation process (S1700) is different from the material properties prediction presentation process (S321) described in Example 1 (Figure 13) in that, after the material properties prediction unit (116) performs prediction, a regression model of regression analysis that is used for the prediction is given to the optimal candidate material properties generation unit (1601), and a process (S1701) of searching for a candidate material that satisfies a predetermined condition, such as the maximum or minimum material properties, and is not included in the material DB (112) is performed using the regression model. In this search, an explanatory variable that satisfies a predetermined condition is searched by an optimum value search method for a known function, such as a gradient descent method, a conjugate gradient method, or a genetic algorithm.

The result of this search is displayed on a material properties prediction result screen (S1702).

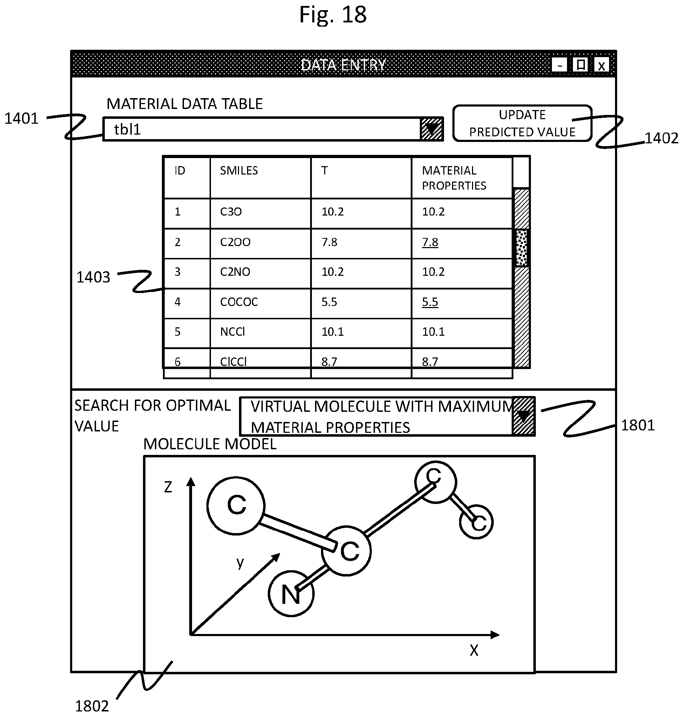

Figure 18 illustrates an example of this displayed result. This screen is different from Example 1 (Figure 14) in that the foregoing searched virtual molecular structure is displayed in a lower portion. This predetermined condition is displayed in a drop-down box (1801), and the user (102) can select one item from this drop-down box (1801). Then, a three-dimensional molecular structure corresponding to the selected result is displayed on a screen (1802). In this display, coordinates of atoms constituting the molecule are calculated by the logic as illustrated in Figure 11 and are used. Lines indicating bonding are drawn on the assumption that the atoms are bonded when distances between the atoms are smaller than the Van der Waals radius. Before this calculation, for example, processing such as evaluation relating to a constraint such as a constraint that the number of atoms bonded to a carbon atom is 4 may be performed.

According to Example 2, a candidate other than a candidate compound given by the user (102) can be selected and it is expected to increase the possibility that a compound that the user has not noticed can be found.

According to the examples described above, to perform prediction based on the spatial structure of a molecule, a feature quantity that has a one-to-one correspondence with the spatial structure of the single molecule is used without allowing freedom with respect to the selection of a coordinate system such that inverse calculation is possible. Therefore, it is possible to predict material properties by incorporating the three-dimensional structure of the molecule into the prediction, which leads to more appropriate screening.

That is, in prediction evaluation for screening of experimental design, it will be possible to perform more accurate prediction by incorporating the three-dimensional structure of a molecule into the prediction. In addition, since the three-dimensional structure of a molecule having a specific predicted value can be inversely calculated, it is possible to estimate the shape of a molecule having desirable properties. As a result, it will be easier to make experimental design, and it will be possible to develop a good material by conducting a small number of experiments.

As described above in the examples, the inventers have paid attention to a problem that, when a feature quantity is used based on the spatial structure of a molecule in order to improve the accuracy of predicting material properties, a coordinate system in the molecule is not uniquely determined, the shape of the molecule cannot be inversely calculated from the feature quantity, and thus the molecule corresponding to an optimal solution is difficult to understand. Therefore, the examples present the methods for selecting the most important three atoms in a molecule as a feature quantity representing the three-dimensional structure of the molecule, and using linear distances from the atoms as the feature quantity. As a result, it is possible to define the spatial structure of a molecule without allowing freedom with respect to the selection of a coordinate system and it is possible to predict material properties based on the three-dimensional structure of the molecule.

### Reference Signs List

- 101: Material properties prediction device
- 113: Three-dimensional molecular structure calculation unit
- 114: Spatial structure feature quantity calculation unit
- 115: Spatial structure feature quantity DB
- S311: Material DB update process
- S321: Material properties prediction presentation process
- S403: Spatial structure feature quantity generation process
- 701: Experiment ID
- 1200: Spatial structure feature quantity table

## Claims

1. A material properties prediction system that predicts properties of a material, comprising:
a three-dimensional molecular structure calculation unit having a function of calculating positional coordinates of atoms constituting a molecule from a structural formula of the material;
a spatial structure feature quantity calculation unit having a function of selecting three atoms to form a triangle based on the positional coordinates of the atoms calculated by the three-dimensional molecular structure calculation unit and calculating, as a spatial structure feature quantity, distances between the three atoms and another atom; and
a material properties prediction unit that predicts the material properties using, as an explanatory variable, the spatial structure feature quantity generated by the spatial structure feature quantity calculation unit.

2. The material properties prediction system according to claim 1, further comprising:
an optimal candidate material properties generation unit having a function of searching for a condition for the spatial structure feature quantity such that the material properties to be predicted by the material properties prediction unit satisfy a predetermined standard, and forming a virtual molecular structure from positional coordinates of atoms satisfying the condition.

3. The material properties prediction system according to claim 1,
wherein the spatial structure feature quantity calculation unit selects, as a standard for selecting the three atoms forming the triangle, a combination forming the triangle having the largest area.

4. The material properties prediction system according to claim 1,
wherein the spatial structure feature quantity calculation unit selects a carbon atom as a standard for selecting the three atoms forming the triangle on a priority basis.

5. The material properties prediction system according to claim 1,
wherein when distances between the three atoms and another atom are to be calculated, the spatial structure feature quantity calculation unit calculates the spatial structure feature quantity based on a direction with respect to a formed surface of the triangle while changing signs to be positive and negative.

6. The material properties prediction system according to claim 1,
wherein the spatial structure feature quantity calculation unit defines a circulation direction of the three atoms to select the three atoms forming the triangle.

7. The material properties prediction system according to claim 1,
wherein the spatial structure feature quantity generated by the spatial structure feature quantity calculation unit is stored as a spatial structure feature quantity database, and
the spatial structure feature quantity database includes information identifying the order of the three atoms forming the triangle, distances between the three atoms forming the triangle, and distances between the three atoms forming the triangle and another atom.

8. An information processing method comprising:
performing a three-dimensional molecular structure calculation process of receiving a structural formula of a material and calculating positions of atoms constituting a molecule from the structural formula of the material; and
performing a spatial structure feature quantity calculation process of selecting three atoms to form a triangle based on the calculated positions of the atoms, and calculating distances between the three atoms and another atom to obtain a spatial structure feature quantity.

9. The information processing method according to claim 8,
wherein a combination forming the triangle having the largest area is selected as a standard for selecting the three atoms forming the triangle.

10. The information processing method according to claim 8,
wherein a carbon atom is selected on a priority basis as a standard for selecting the three atoms forming the triangle.

11. The information processing method according to claim 8,
wherein a circulation direction of the three atoms is defined to select the three atoms forming the triangle.

12. The information processing method according to claim 11,
wherein when distances between the three atoms and another atom are to be calculated, the spatial structure feature quantity is calculated based on a direction with respect to a formed surface of the triangle while signs are changed to be positive and negative.

13. The information processing method according to claim 8,
wherein the spatial structure feature quantity calculated in the spatial structure feature quantity calculation process is stored as a spatial structure feature quantity database, and
the spatial structure feature quantity database includes information identifying the order of the three atoms forming the triangle, distances between the three atoms forming the triangle, and distances between the three atoms forming the triangle and another atom.

14. The information processing method according to claim 8, further comprising performing a material properties prediction process of predicting the material properties using, as an explanatory variable, the spatial structure feature quantity calculated in the spatial structure feature quantity calculation process.

15. The information processing method according to claim 14,
wherein the material properties prediction process uses a prediction function to perform the prediction, and the prediction function is obtained by performing regression analysis using the spatial structure feature quantity as the explanatory variable and the material properties as an objective variable.
